(19) Europäisches Patentamt
European Patent Office
Office européen des brevets

(11) Publication number: **0 175 814**
**B1**

(12) **EUROPEAN PATENT SPECIFICATION**

(45) Date of publication of patent specification: **23.05.90**

(51) Int. Cl.[5]: **C 07 D 501/06**

(21) Application number: **84112657.6**

(22) Date of filing: **19.10.84**

(54) **Process for preparing cephem derivatives.**

(30) Priority: **27.09.84 KR 845935**

(43) Date of publication of application:
**02.04.86 Bulletin 86/14**

(45) Publication of the grant of the patent:
**23.05.90 Bulletin 90/21**

(84) Designated Contracting States:
**AT CH DE FR GB IT LI**

(56) References cited:
**EP-A-0 002 774**

(73) Proprietor: **HAN-MI PHARMA IND. CO., LTD.**
**822-4, Yoksam-dong**
**Kangnam-ku Seoul (KR)**

(72) Inventor: **Lim, Sung Ki**
**525, Changsin-dong**
**Chongro-ku Seoul (KR)**
Inventor: **Moon, Soon Ku**
**318-175, Mok-dong**
**Kanseo-ku Seoul (KR)**
Inventor: **Lee, Gwan Sun**
**No. 81-201 Karak Apt. Karak-dong**
**Kangdong-ku Seoul (KR)**

(74) Representative: **Kraus, Walter, Dr. et al**
**Patentanwälte Kraus, Weisert & Partner**
**Thomas-Wimmer-Ring 15**
**D-8000 München 22 (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

Field of the Invention

This invention relates to an improvement in relation to a process for preparing a cephem compound of the formula (I)

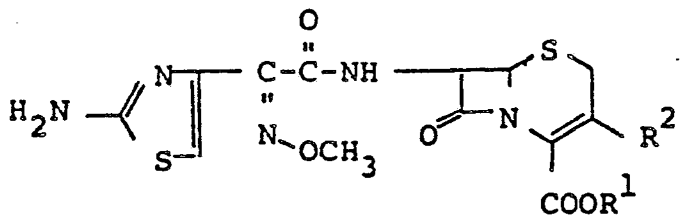

(I)

wherein

$R^1$ is hydrogen or metal salt,

$R^2$ is hydrogen, halogen, acetoxymethyl or —$CH_2$—S—$R^3$ in which $R^3$ is 5- or 6-membered heterocyclic ring having 1—4 heteroatoms optionally substituted by lower alkyl.

More particularly it relates to a novel improved process for preparing the compound of the formula (I) by reacting 2-(2-aminothiazol-4-yl)-2-syn-methoxyiminoacetic acid in the presence of 4-dimethylaminopyridine, the catalyst and a condensing agent with 1-hydroxybenzotriazole to obtain a solvate of a reactive amide thereof with solvent used and when by reacting the obtained reactive amide with 7-aminocephem derivative to obtain the compound of the formula (I).

Description of the Prior Art

Various methods of preparing a semisynthetic β-lactam antibiotics were reported in various literatures and patents. That is, various β-lactam antibiotics were prepared by condensing derivatives of 7-aminodeoxycephalosporanic acid (7—ADCA) or of 7-aminocephalosporanic acid (7—ACA) with reactive derivatives of organic acids to form peptide linkages and obtain the β-lactam antibiotics.

There are several methods for preparing the compound of the formula (I). For examples, the amino-protected organic acid of the formula (II) introduced by R-group,

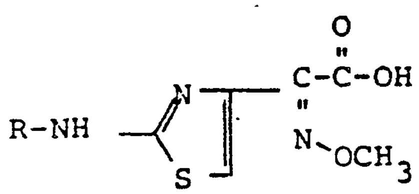

(II)

wherein

R is an amino protecting group,

is converted into a reactive derivatives thereof and the reactive derivatives are used as acylating agents for 7—ACA, 7—ADCA or derivatives thereof. Reactive derivative as such acid halide, reactive ester or reactive amide is usually used.

Various methods for preparing acid halide of the organic acid (II) and use of the acid halide as acylating agent are disclosed in Japanese Patent Laid Open Publication Nos. 102293/1977, 34795/1978, 68796/1978, 52096/1979 and 157596/1979 and British Patent No. 2025933. According to the above methods, organic acid(II) is converted by using thionyl chloride($SOCl_2$), phosphorus pentachloride($PCl_5$) or phosphorus oxychloride($POCl_3$) into acid halide thereof, then 7—ADCA or 7—ACA is acylated with the acid halide to obtain acylated product and then protecting group R is removed from the acylated product to obtain the compound of the formula (I).

However, not only the reaction conditions of the said method is complicated but amino protecting and deprotecting steps are required. Moreover, acid halide is unstable.

Methods for preparing a compound of formula (I) by synthesizing a reactive ester of organic acid (II) and by acylating 7—ACA, 7—ADCA or derivatives thereof with the reactive ester are disclosed in Japanese Pagent Laid Open Publication Nos. 102293/1977, 95993/1979 and 152488/1981.

According to the methods, 2-pyridine thioester, 2-benzothiazol ester or 1-hydroxybenzotriazol ester of the compound (II) is first prepared and then 7—ACA, 7—ADCA or derivatives thereof is acylated with the said esters to obtain the compound of the formula (I). However, the side reaction arises at the time of preparing ester and the acylation should be carried out for a long time with low yield. Though there are also acid amide or mixed anhydride methods for preparing compound (I), those methods also have such problems as mentioned above. In the above methods, after the amino group of 2-(2-aminothiazolyl-4-yl)-2-

syn-methoxy iminoactive acid is first protected by introducing R group, reactive derivative such as acid halide or reactive ester, etc., is prepared. Then, acylation is carried out and the protecting group is removed.

The method of using 1-hydroxybenzotriazole ester and amide of compound (II) is disclosed in Japanese Patent Laid Open Publication No. 95593/1979. According to this method, an ester of formula (VII) and amide of formula (VIII) are produced almost in the same ratio.

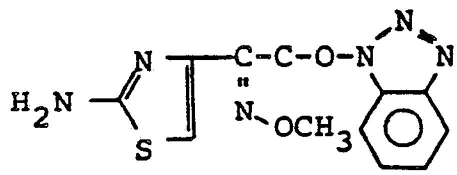
(VII)

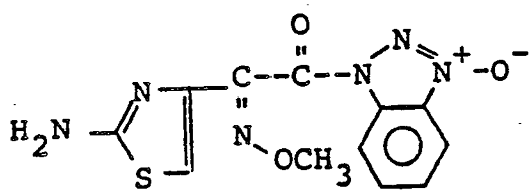
(VIII)

However, the reactive ester of the formula (VII) is very unstable to decompose into acid of formula (II) and 1-hydroxybenzotriazole. Therefore, there exists much difficulties to separate the use the products as acylating agents.

Summary of the Invention

It has been unexpectedly and surprisingly found that when an organic acid of formula (II) wherein R is hydrogen is reacted in the presence of catalytic amount of 4-dimethylaminopyridine (V) and condensing agent with 1-hydroxybenzotriazole of formula (IV) in certain organic solvent a solvate of a reactive amide of formula (VIII) which is defined hereinafter can be obtained selectively in high yield and the solvate of the reactive amide (VIII) can be reacted at ambient temperature with a compound of formula (III) which is defined hereinafter and isolated easily to obtain a compound of formula (I) in high yield.

Detailed Description of the Invention

This invention relates to a novel process for preparing a compound of formula (I)

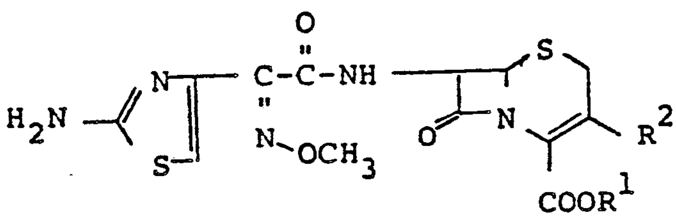
(I)

wherein

$R^1$ is hydrogen or metal salt,

$R^2$ is hydrogen, halogen, acetoxymethyl or $-CH_2-S-R^3$ in which $R^3$ is 5- or 6-membered heterocyclic ring having 1—4 hetero atoms optionally substituted by lower alkyl,

by reacting a compound of formula (II)

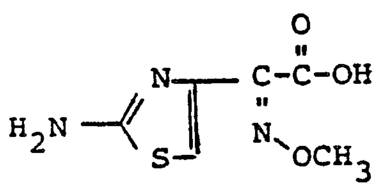
(II)

in the presence of catalytic amount of 4-dimethylaminopyridine and condensing agent with a compound of formula (IV)

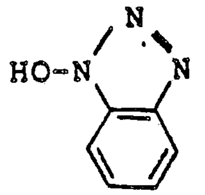

(IV)

in certain organic solvent to obtain a solvate of the compound of formula (VIII) with solvent used and then reacting a compound of formula (III) at an ambient temperature with the obtained solvate of the compound of formula (VIII) in very high yield.

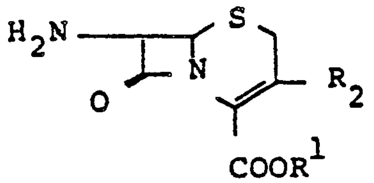

(III)

wherein
$R^1$ and $R^2$ are defined above.
The object of this invention can be prepared by the following processes:

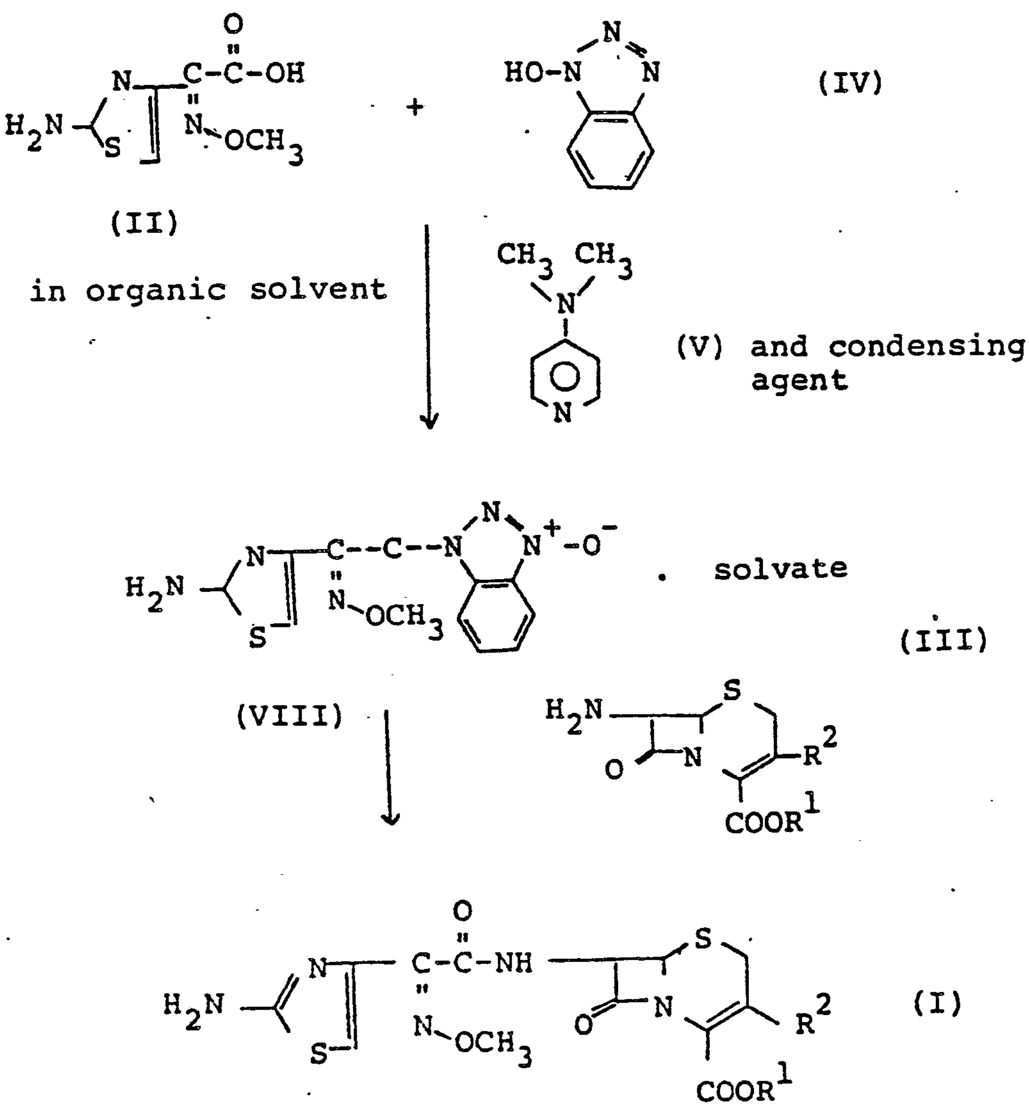


(II) (IV) (V) (VIII) (III) (I)

wherein
$R^1$, $R^2$ and $R^3$ are as defined above.

Halo represented by $R^2$ means bromo or chloro.

Examples of 5- or 6-membered heterocyclic ring having 1—4 hetero atoms optionally substituted by lower alkyl defined by $R^3$ in $—CH_2—S—R^3$ represented by $R^2$ are 1,2,3-triazolyl, 5-methyl-1,3,4-thiadiazolyl, 1-methyl-1H-tetrazol-5-yl and 2,5-dihydro-2-methyl-6-hydroxy-5-oxo-as-triazin-3-yl, etc.

The inventors found out when the compound of formula (II) is reacted in a solvent such as N,N,-dimethylformamide(DMF), N,N-dimethylacetamide(DMA) or N-methyl-2-pyrollidone(NMP) with the compound of formula (IV) in the presence of the compound of formula (V) and condensing agent, the compound of formula (VIII) is selectively obtained as solvate of the compound of formula (VIII) with solvent used and the solvate can be reacted homogeneously with the compound of formula (III) in the next acylation step to obtain the compound of formula (I) in very high yield.

Condensing agent which can be used in this invention is conventional ones such as dicyclohexylcarbodiimide, N-cyclohexyl-N'-morphorinoethylcarbodiimide, N-ethyl-N'-(3-dimethylaminopropyl)carbodiimide and 1-(p-chlorobenzenesulfonyloxy)-6-chloro-1H-benzotriazol. The most preferred one is dicyclohexylcarbodiimide. Condensing agent can be used in the amount of about 0.1—2.0 moles to one mole of the organic acid (II). About equimolar amount of the condensing agent is preferable.

The catalyst(V) can be preferably used in the amount of about 0.5—5 weight percent to the organic acid(II). Reaction is usually carried out at ambient temperature in about 1—1.5 hours.

Then, the reactive amide (VIII) is produced nearly quantitatively as solvate form which is more reactive and can be easily isolated as precipitate. The solid precipitated is filtered out from the reaction mixture.

Then, acylation reaction is preferably carried out in mixed solvent of water solution of a base and organic solvent. Examples of base which can be used in this reaction are inorganic base such as sodium hydrogen carbonate, potassium hydrogen carbonate, sodium carbonate, potassium carbonate, etc. or organic base such as trimethylamine, or triethylamine. Sodium hydrogen carbonate is preferably used.

Organic solvent which can be used in this process is acetonitrile, acetone, ethyl acetate, tetrahydrofuran, N,N-dimethylformamide, N,N-dimethylacetamide or dioxan, etc. The preferred ones are acetonitrile, acetone, tetrahydrofuran and N,N-dimethyformamide.

In processes of this invention, the compound of formula (III) is dissolved in a basic solution and organic solvent is added thereto. The solvate of the reactive amide (VIII) is added as crystalline form to the mixture and the mixture is stirred at ambient temperature for about 1—5 hours. The reaction proceeds quantitatively. Then organic solvent is distilled in vacuo. Water layer obtained is adjusted to about pH 4.0 to separate 1-hydroxybenzotriazole produced during the reaction and then, after the water layer is saturated with sodium chloride, ethyl acetate is slowly added to the saturated water layer with stirring and pH is adjusted to isoelectric point to obtain the compound of formula (I) as precipitates.

About 10—30 times amount of water and organic solvent to that of the compound of formula (III) is preferably used. The ratio of water and organic solvent is preferable from 2:1 to 1:2.

This invention has the following advantages compared with known methods:

1. By using small amount of catalyst, a reactive amide (VIII) is obtained in very high yield nearly quantitatively as solvate form with solvent used which is very reactive and stable.

2. The solvate of the reactive amide (VIII) is very soluble in various organic solvent, the acylation by this solvate is proceeded homogeneously in short reaction time.

3. Unlike acid halide or mixed anhydride, the solvate of the reactive amide (VIII) is very stable at ambient temperature and therefore the solvate of the reactive amide (VIII) is available in market.

4. The process can be carried out at ambient temperature.

5. Even though the amino group of the organic acid compound (II) is not protected, the reaction proceeds quantitatively without any side reaction. Therefore, there is no need for protecting and deprotecting the amino group of the compound (II).

6. When the reaction is completed, the object compound can be easily separated from the reaction solution.

Accordingly, these processes can be carried out in simple manufacturing facilities and have no complicated procedures compared with the known methods. So, this invention provides a novel and economical manufacturing process for preparing the compound of formula (I).

The following examples are given only for the purpose of illustrating this invention in more detail.

Example 1

10.05 g of 2-(2-aminothiazol-4-yl)-2-syn-methoxyiminoacetic acid and 6.76 g of 1-hydroxybenzotriazole were completely dissolved in 100 ml of N,N-dimethylformamide, 0.3 g of 4-dimethylaminopyridine was added thereto and the resulting solution was stirred at normal temperature for 10 minutes. To the resulting solution was dropped a solution of 10.3 g of dicyclohexylcarbodiimide in 30 ml of N,N-dimethylformamide during 10 minutes. The resulting mixture was stirred at normal temperature for 1 hours to form precipitates.

The precipitate was filtered out and washed with N,N-dimethylformamide. The filtrate and washings were combined and stirred at normal temperature for 2 hours and at 0°C for 2 hours successively to form precipitate. The precipitate was washed with N,N-dimethylformamide and dried to give DMF solvate of 1-[α-syn-methoxyimino-α-(2-aminothiazol-4-yl)-acetyl]-benzotriazol-3-oxide, the reactive amide. Yield: 17.6 g (90%)

IR (KBr $cm^{-1}$): 1730 (amide carbonyl).

NMR (DMSO-$d_6$, δ, ppm): 2.27 (3H, s, —N—$CH_3$), 2.90 (3H, s, —N—$CH_3$), 3.90 (3H, s, —$OCH_3$), 7.07 (1H, s, thiazol-H), 7.13 (2H, s, —$NH_2$), 7.5—8.5 (4H, m, benzotriazol-H), 7.97 (1H, s, H—C(=O)—)

mp: 156—159 (decomp.).

Example 2

Reaction was carried out in a similar manner to that of the example 1 by using N-methyl-2-pyrollidone as solvent in place of N,N-dimethylformamide to give NMP solvate of 1-[α-syn-methoxyimino-α-(2-aminothiazol-4-yl)-acetyl]-benzotriazol-3-oxide. Yield: 17.72 g (85%) with same IR and NMR data as in example 1.

Example 3

Reaction was carried out in a similar manner to that of example 1 by using N-cyclohexyl-N'-morphorinoethylcarbodiimide as condensing agent in place of dicyclohexylcarbodiimide to give DMF solvate of 1-[α-syn-methoxyimino-α-(2-aminothiazol-4-yl)-acetyl]-benzotriazol-3-oxide. Yield: 17.2 g (88%) with same IR and NMR data in example 1.

Example 4

10.05 g 2-(2-aminothiazol-4-yl)-2-syn-methoxyiminoacetic acid and 6.76 g of 1-hydroxybenzotriazole were dissolved in 100 ml of N,N-dimethylacetamide. 0.3 g of 4-dimethylaminopyridine was added to the solution and the solution was stirred for 10 minutes. To the solution, a solution of 10.3 g of N,N-dicyclohexycarbodiimide in 30 ml of N,N-dimethylacetamide was dropped during 10 minutes and the mixture was stirred at normal temperature for 10 minutes and the precipitates formed was filtered out. The precipitate was washed with N,N-dimethylacetamide. The filtrate and washings were combined and stirred at normal temperature for 2 hours and at 0°C for 2 hours successively to form precipitate. The precipitate was filtered and dried to give DMA solvate of 1-[α-syn-methoxyimino-α-(2-aminothiazol-4-yl)-acetyl]-benzotriazol-3-oxide. Yield: 18.2 g (88%).

IR (KBr, $cm^{-1}$): 1730 (amide carbonyl), 1655 (DMA carbonyl).

NMR (DMSO-$d_6$, δ, ppm): 2.05 (3H, s, $CH_3$—C(=O)) 2.30 (3H, s —N —$CH_3$), 2.95 (3H, s, —N—$CH_3$), 3.95 (3H, s, —$OCH_3$), 7.10 (1H, s, thiazol-H), 7.20 (2H, s, —$NH_2$), 7.4—8.4 (4H, m, benzotriazol-H).

mp: 157—160°C (decomp.).

Example 5

5.44 g of 7-aminocephalosporanic acid were added to mixture of 100 ml of water and 200 ml of acetonitrile. To mixture were added 2.4 g of sodium carbonate and the mixture was stirred at room temperature for 10 minutes to dissolve completely. To resulting mixture were added 7.87 g of DMA solvate of 1-[α-syn-methoxyimino-α-(2-aminothiazol-4-yl)-acetyl]-benzotriazol-3-oxide prepared in example 1 and the mixture was stirred at normal temperature for 4 hours to complete reaction. Acetonitrile was distilled off in vacuo from the mixture. The solution was chilled to 0—5°C, adjusted to pH 4.0 with 2N—HCl and stirred for 1 hour to form precipitate. After mixture was filtered, filtrate was saturated with sodium chloride and 120 ml of ethyl acetate was added thereto and pH was adjusted with stirring vigorously to 2.5 with 2N—HCl to form pale yellow precipitate. After stirred for 1 hour, filtered and dried to give 7-[[2-(2-aminothiazol-4-yl)-2-syn-methoxyimino]acetamido]cephalosporanic acid(cefotaxime). Yield 8.65 g (95%).

IR (KBr, $cm^{-1}$): 1780 (α-lactamcarbonyl).

NMR ($D_2O$/$NaHCO_3$, δ, ppm): 2.08 (3H, s, —C(=O)—$CH_3$) 3.53 (2H, d, $C_2$—H), 4.02 (3H, s, —$OCH_3$), 4.84 (2H, d, 3—$CH_2$—), 5.20 (1H, d, $C_6$—H), 5.82 (1H, d, $C_7$—H), 6.97 (1H, s, thiazol-H).

Example 6

Reaction was carried out in a similar manner to that of example 5 by using tetrahydrofuran in place of acetonitrile to give 7-[[2-(2-aminothiazol-4-yl)-2-syn-methoxyimino]acetamido]cephalosporanic acid. Yield: 8.44 g (93%).

Example 7

Reaction was carried out in a similar manner to that of example 5 by using acetone in place of acetonitrile to give 7-[[2-(2-aminothiazol-4-yl)-2-syn-methoxyimino]acetamido]cephalosporanic acid. Yield: 8.26 g (91%) with same IR and NMR data with example 5.

Example 8

5.44 g of 7-aminocephalosporanic acid were added to mixture of 100 ml of water and 40 ml of N,N-dimethylformamide and 2.4 g of sodium hydrogen carbonate were added thereto. The mixture was stirred at room temperature for 10 minutes to be dissolved and chilled below 10°C. Solution of 7.87 g of DMF solvate of 1-[α-syn-methoxyimino-α-(2-aminothiazol-4-yl)-acetyl]-benzotriazol-3-oxide dissolved in 40 ml of N,N-dimethylformamide were dropped thereto during 10 minutes. The solution was stirred at room temperature for 4 hours to complete reaction. After N,N-dimethylformamide was extracted with 150 ml of ethyl acetate, water-layer was saturated with sodium chloride. The solution was chilled to 5°C, adjusted to pH 4.0 with 2N—HCl and filtered to eliminate impurities. By adjusting the pH of the filtrate to 2.5 with 2N—HCl to obtain pale yellow precipitate. After the mixture was stirred for 1 hour, filtered. Precipitate was washed with water and dried to give 7-[[2-(2-aminothiazol-4-yl)-2-syn-methoxyimino]acetamido]cephalosporanic acid. Yield: 7.98 g (88%) with same IR and NMR data in example 5.

Example 9

Reaction was carried out in similar manner to that of example 8 by using N,N-dimethylacetamide in place of N,N-dimethylformamide to give 7-[[2-(2-aminothiazol-4-yl)-2-syn-methoxyimino]acetamido]-cephalosporanic acid. Yield: 7.96 g (88%) with same IR and NMR data in example 5.
example 5.

Example 10

Reaction was carried out for 3 hours in similar manner to that of example 5 by using 8.10 g of DMA solvate prepared in Example 4 to give 7-[[2-(2-aminothiazol-4-yl)-2-syn-methoxyimino]acetamido]-cephalosporanic acid. Yield: 8.92 g (98%) with same IR and NMR data in example 5.

Example 11

3.28 g of 7-amino[3-(1-methyl-1H-tetrazol-5-yl)-thiomethyl]-3-cephem-4-carboxylic acid were added to 35 ml of water. 2.0 g of sodium hydrogen carbonate were added thereto. After mixture was stirred at room temperature to be dissolved, 40 ml of acetonitrile was added thereto and the mixture was chilled to 10°C. 4.68 g of DMF solvate of 1-[α-syn-methoxyimino-α-(2-aminothiazol-4-yl)-acetyl]-benzotriazol-3-oxide prepared in example 1 were added thereto. When the mixture was stirred at room temperature for 5 hours, the mixture was dissolved and the reaction was completed. To water layer obtained by distilling acetonitrile in vacuo was added another 40 ml of water. The mixture was adjusted to pH 3.8 with 2N—HCl, stirred for 1 hour and filtered to eliminate precipitate. Filtrate was saturated with sodium chloride, adjusted to pH 2.7 with 2N—HCl to give precipitate. The mixture was stirred at 5°C for 2 hours, filtered and dried to give 7-[[2-(2-aminothiazol-4-yl)-2-syn-methoxyimino]acetamido]-3-[[3-(1-methyl-1H-tetrazol-5-yl)thiomethyl]-3-cephem-4-carboxylic acid(cefmenoxime.). Yield: 4.65 g (91%).

IR (KBr, $cm^{-1}$): 1780 (β-lactamcarbonyl).

NMR ($D_2O/NaHCO_3$, δ): 3.84 (2H, d, $C_2$—H), 4.01 (3H, s, —$OCH_3$), 4.05 (3H, s, =N—$CH_3$), 5.20 (1H, d, $C_6$-H), 5.77 (1H, d, $C_7$—H), 7.01 (1H, s, thiazol-H).

Example 12

Reaction was carried out in a similar manner to that of example 10 by using 40 ml of ethyl acetate in place of acetonitrile to give 7-[[2-(2-aminothiazol-4-yl)-2-syn-methoxyimino]acetamido]-3-[(1-methyl-1H-tetrazol-5-yl)thiomethyl]-3-cephem-4-carboxylic acid. Yield: 4.6 g (90%) with same IR and NMR data as in example 10.

Example 13

Reaction was carried out for 3 hours in similar manner to that of example 11 by using 4.05 g of DMA solvate prepared in example 4 to give 7-[2-(2-amino-1H-tetrazol-5-yl)thiomethyl]-3-cephem-4-carboxylic acid. Yield: 4.97 g (94%) with same IR and NMR data as in example 11.

Example 14

3.71 g of 7-amino-3-(2,5-dihydro-2-methyl-6-hydroxy-5-oxo-as-triazin-3-yl)thiomethyl-3-cephem-4-carboxylic acid were added to mixture of 40 ml water and 30 ml of tetrahydrofuran and 2.79 of sodium hydrogen carbonate were added thereto obtain clear solution of pH 8.0—8.2. 4.68 g of DMF solvate of 1-[α-syn-methoxyimino-α-(2-aminothiazol-4-yl)-acetyl]-benzotriazol-3-oxide were added thereto and the mixture was stirred at 5°C for 1 hour and then after raising the temperature to 25°C, the mixture was stirred for 5 hours. Tetrahydrofuran was distilled off in vacuo from the solution, 30 ml of water was added thereto and the solution was adjusted to pH 4.2 with 2N—HCl, stirred for 30 minutes and filtered to eliminate insoluble matters formed. Filtrate was saturated with sodium chloride, adjusted to pH 2.9—3.0 with 2N—HCl to give precipitate. The mixture was stirred with maintaining same pH for 2 hours, filtered and dried to give 7-[[2-(2-aminothiazol-4-yl)-2-syn-methoxyimino]acetamido]-3-[(2,5-dihydro-6-hydroxy-2-methyl-5-oxo-as-triazine-3-yl)thiomethyl]-3-cephem-4-carboxylic acid. Yield: 4.70 g (85%).

IR (KBr, $cm^{-}$): 1780 (β-lactam carbonyl).

NMR ($D_2O/NaHCO_3$, δ): 3.20 (2H, d, $C_2$—H), 3.62 (3H, s, =N—$CH_3$), 3.95 (3H, s, —$OCH_3$), 4.21 (2H, d, —S—$CH_2$—), 5.17 (1H, d, $C_6$—H), 5.72 (1H, d, $C_7$—H), 6.95 (1H, s, thiazol-H).

Example 15

Reaction was carried out in a similar manner to that of example 12 by using 40 ml of acetonitrile in place of tetrahydrofuran to give 7-[[2-(2-aminothiazol-4-yl)-2-syn-methoxyimino]acetamido]-3-[(2,5-dihydro-6-hydroxy-2-methyl-5-oxo-as-triazine-3-yl)thiomethyl]-3-cephem-4-carboxylic acid. Yield: 4.50 g (81%) with same IR and NMR data as in example 12.

Example 16

Reaction was carried out for 4 hours in similar manner to that of example 14 by using 4.05 g of DMA solvate prepared in example 4 to give 7-[[2-(2-aminothiazol-4-yl)-2-syn-methoxyimino]acetamido]-3-[(2,5-dihydro-6-hydroxy-2-methyl-5-oxo-as-triazine-3-yl)thiomethyl]-3-cephem-4-carboxylic acid. Yield: 5.27 g (91%) with same IR and NMR data as in example 14.

Example 17

1.88 g of 7-amino-3-cephem-4-carboxylic acid were added to mixture of 20 ml water and 20 ml of tetrahydrofuran and 2.80 g of sodium hydrogen carbonate were added thereto. To the solution chilled to 5°C, were added 3.91 g of DMF solvate of 1-[α-syn-methoxyimino-α-(2-aminothiazol-4-yl)-acetyl]-benzotriazol-3-oxide during 10 minutes. The mixture was stirred at 5°C for 1 hour and 25°C for 2 hours. After tetrahydrofuran was distilled off in vacuo, 15 ml of water was added thereto and the mixture was chilled to 5°C, the solution was adjusted to pH 2.4 with 2N—HCl, stirred for 30 minutes and filtered to eliminate insoluble matters formed. Filtrate was saturated with sodium chloride, adjusted to pH 2.8 with 2N—HCl to form precipitate. The mixture was stirred with maintaining same pH for 2 hours under ice-cooling and filtered. The precipitate was washed with saturated sodium chloride solution and chilled water in turn and dried to give 7-[[2-(2-aminothiazol-4-yl)-2-syn-methoxyimino]acetamido]-3-cephem-4-carboxylic acid. Yield: 3.48 g (91%).

IR (KBr, $cm^{-}$): 1780, 1695, 1655.

NMR (DMSO-$d_6$, δ): 3.60 (2H, broad, $C_2$—H), 3.84 (3H, s, —$OCH_3$), 5.12 (1H, d, $C_6$—H), 5.84 (1H, d, $C_7$—H), 6.52 (1H, s, 3—H), 6.76 (1H, s, thiazol-H), 7.26 (2H, broad, —$NH_2$), 9.65 (1H, d, —NH—CO).

Example 18

Reaction was carried out in a similar manner to that of example 14 by using 20 ml of acetone in place of tetrahydrofuran to give 7-[[2-(2-aminothiazol-4-yl)-2-syn-methoxyimino]acetamido]-3-cephem-4-carboxylic acid. Yield: 3.40 g (90%) with same IR and NMR data as in example 14.

Example 19

Reaction was carried out for 3 hours in similar manner to that of example 17 by using 4.05 g of DMA solvate prepared in example 4 to give 7-[[2-(2-aminothiazol-4-yl)-2-syn-methoxyimino]acetamido]-3-cephem-4-carboxylic acid. Yield: 5.27 g (94%) with same IR and NMR data as in example 17.

**Claims**

1. A process for preparing a compound of formula (I)

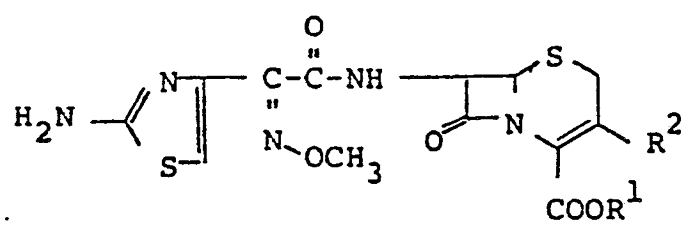

(I)

wherein

$R^1$ is hydrogen or metal salt, and

$R^2$ is hydrogen, halogen, acetoxymethyl or —CH—S—$R^3$ in which $R^3$ is 5- or 6-membered heterocyclic ring having 1—4 heteroatoms optionally substituted by lower alkyl, by reacting a compound of formula (II)

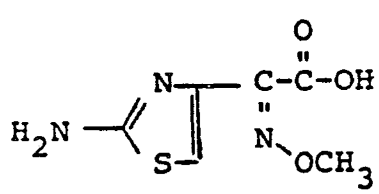

(II)

in the presence of catalytic amount of the compound of formula (V)

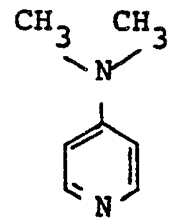

(V)

and a condensing agent with 1-hydroxybenzotriazole of formula (IV)

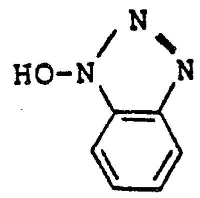

(IV)

to give a solvate of a reactive amide of formula (VIII) with solvent used,

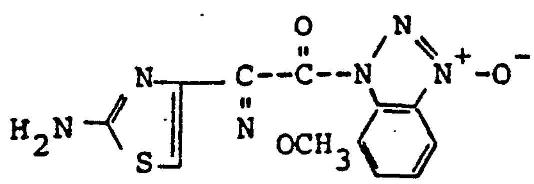

. solvate (VIII)

and then by acylating a compound of formula (III)

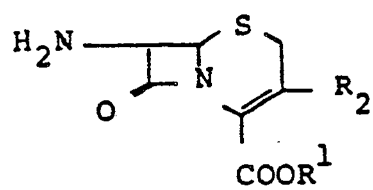

(III)

wherein

$R^1$ and $R^2$ are defined above, with the solvate of the reactive amide of the formula (VIII) to obtain the compound of formula (I).

2. A process according to claim 1 in which a solvate of the reactive amide of formula (VIII) is prepared from solvent selected from the group consisted of N,N-dimethylformamide, N,N-dimethyl acetamide and N-methyl-2-pyrollidone.

3. A process according to claim 1 or 2 in which a condensing agent is selected from the group consisted of N,N-dicyclohexylcarbodiimide, N-cyclohexyl-N'-morphorinoethylcarbodiimide, N-ethyl-N'-(dimethyl aminopropyl)carbodiimide or 1-(p-chlorobenzenesulfonyloxy-6-chloro-1H-benzotriazole.

4. A process according to claim 1, 2 or 3 in which acylation is carried out at ambient temperature and in mixed solvent of water and an organic solvent selected from the group consisted of acetonitrile, acetone, tetrahydrofuran, N,N-dimethylformamide, N,N-dimethylacetamide or mixture thereof.

**Patentansprüche**

1. Verfahren zur Herstellung einer Verbindung der Formel (I)

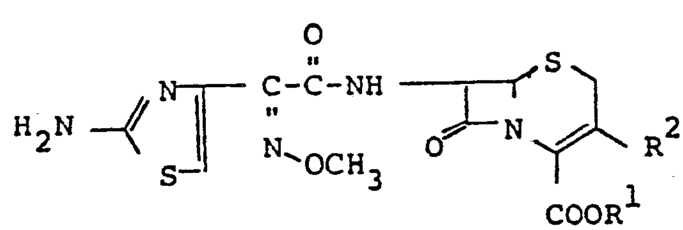

(I)

worin

$R^1$ Wasserstoff oder ein Metallsalz ist und $R^2$ für Wasserstoff, Halogen, Acetoxymethyl oder —CH—S—$R^3$ steht, worin $R^3$ ein gegebenenfalls durch Niedrigalkyl substituierter 5- oder 6gliedriger heterocyclischer Ring mit 1 bis 4 Heteroatomen ist, dadurch gekennzeichnet, daß man eine Verbindung der Formel (II)

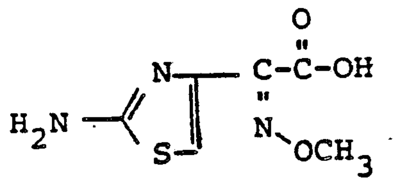

(II)

in Gegenwart einer katalytischen Menger der Verbindung der Formel (V)

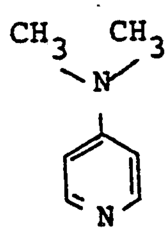

(V)

und eines Kondensationsmittels mit 1-Hydroxybenzotriazol der Formel (IV)

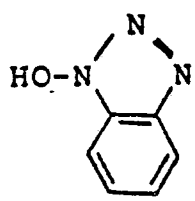

(IV)

umsetzt, um ein Solvat eines reaktiven Amids der Formel (VIII) mit dem verwendeten Lösungsmittel zu erhalten,

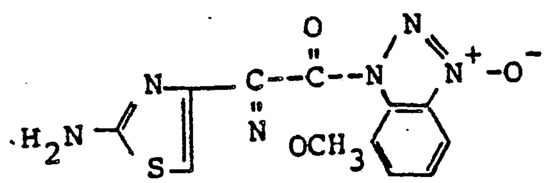

solvate (VIII)

und daß man sodann eine Verbindung der Formel (III),

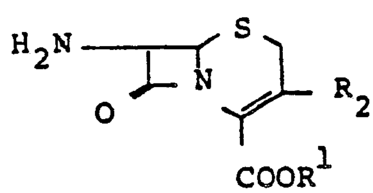

(III)

worin

$R^1$ und $R^2$ wie oben definiert sind, mit dem Solvat des reaktiven Amids der Formel (VIII) unter Erhalt der Verbindung der Formel (I) acyliert.

2. Verfahren nach Anspruch 1, dadurch gekennzeichnet, daß man ein Solvat des reaktiven Amids der Formel (VIII) aus einem Lösungsmittel, ausgewählt aus der Gruppe N,N-Dimethylformamid, N,N-Dimethylacetamid und N-Methyl-2-pyrrolidon, herstellt.

3. Verfahren nach Anspruch 1 oder 2, dadurch gekennzeichnet, daß das Kondensationsmittel aus der Gruppe N,N-Dicyclohexylcarbodiimid, N-Cyclohexyl-N'-morpholinoethylcarbodiimid, N-Ethyl-N'-(dimethylaminopropyl)carbodiimid oder 1-(p-Chlorbenzosulfonyloxy-6-chlor-1H-benzotriazol ausgewählt ist.

4. Verfahren nach Anspruch 1, 2 oder 3, dadurch gekennzeichnet, daß man die Acylierung bei Umgebungstemperatur und in einem Mischlösungsmittel aus Wasser und einem organischen Lösungsmittel, ausgewählt aus der Gruppe Acetonitril, Aceton, Tetrahydrofuran, N,N-Dimethylformamid, N,N-Dimethylacetamid oder einem Gemisch davon, durchführt.

**Revendications**

1. Procédé pour préparer un composé de formule (I)

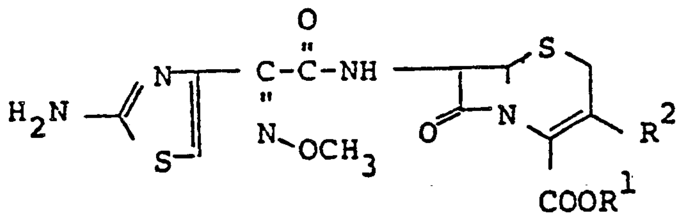

(I)

dans laquelle

$R^1$ est un atome d'hydrogène ou un sel métallique et $R^2$ est un atome d'hydrogène, d'halogène, un groupe acétoxyméthyle ou $—CH—S—R^3$ dans lequel $R^3$ est un noyau hétérocyclique à 5 ou 6 chaînons ayant 1—4 hétéroatomes éventuellement substitués par un groupe alkyle inférieur, caractérisé en ce qu'il comprend la réaction d'un composé de formule (II)

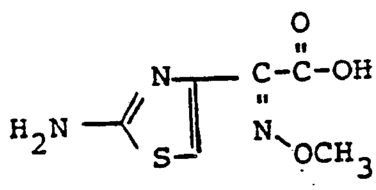

(II)

en présence d'une quantité catalytique du composé de formule (V)

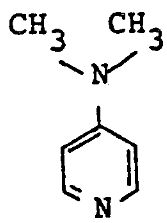

(V)

et d'un agent de condensation, avec un 1-hydroxybenzotriazole de formule (IV)

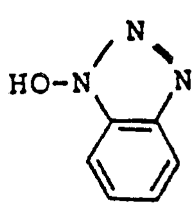

(IV)

pour donner un solvate d'un amide réactif de formule (VIII) avec le solvant mis en ouevre

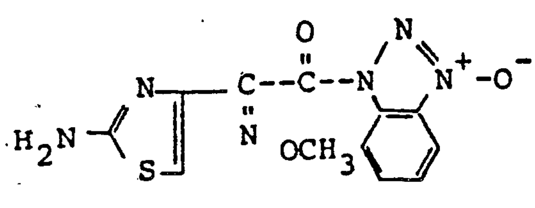

solvate (VIII)

et ensuite l'acylation d'un composé de formule (III)

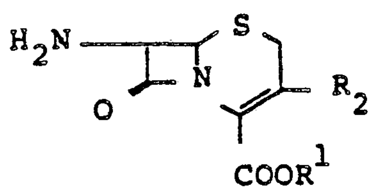

(III)

dans laquelle
$R^1$ et $R^2$ sont tels que définis plus haut, avec le solvate de l'amide réactif de formule (VIII) pour obtenir le composé de formule (I).

2. Procédé suivant la revendication 1, caractérisé en ce qu'un solvate de l'amide réactif de formule (VIII) est préparé à partir d'un solvant choisi parmi le N,N-diméthylformamide, le N,N-diméthylacétamide et le N-méthyl-2-pyrrolidone.

3. Procédé suivant la revendication 1 ou la revendication 2, caractérisé en ce que l'agent de condensation est choisi parmi le N,N-dicyclohexylcarbodiimide, le N-cyclohexyl-N'-morpholinoéthylcarbodiimide, le N-éthyl-N'-(diméthylaminopropyl)-carbodiimide ou le 1-(p-chlorobenzènesulfonyloxy-6-chloro-1H-benzotriazole.

4. Procédé suivant les revendications 1, 2 ou 3, caractérisé en ce que l'acylation est effectuée à température ambiante et dans un mélange solvant d'eau et d'un solvant organique choisi dans le groupe comprenant l'acétonitrile, l'acétone, le tétrahydrofuranne, le N,N-diméthylformamide, le N,N-diméthylacétamide ou leurs mélanges.